(19) Europäisches Patentamt
European Patent Office
Office européen des brevets

(11) Publication number: **0 010 855**
**A1**

(12) **EUROPEAN PATENT APPLICATION**

(21) Application number: **79301986.0**

(22) Date of filing: **25.09.79**

(51) Int. Cl.³: **C 07 C 21/04**, C 07 C 17/34

(30) Priority: **26.10.78 GB 4198778**

(43) Date of publication of application: **14.05.80**
**Bulletin 80/10**

(84) Designated Contracting States: **CH DE FR GB IT NL**

(71) Applicant: **IMPERIAL CHEMICAL INDUSTRIES LIMITED, Imperial Chemical House Millbank, London SW1P 3JF (GB)**

(72) Inventor: **Crosby, John, 12 Oakwood Court Bowdon, Altrincham Cheshire, WA14 3DJ (GB)**
Inventor: **Milner, David John, 18 Eight Acre Whitefield, Manchester, M25 7LW (GB)**

(74) Representative: **Clark, Peter Frederick et al, Imperial Chemical Industries Limited Legal Department, Patents Thames House North Millbank, London SW1P 4QG (GB)**

(54) **Process for the manufacture of 1,2-dichloro-3,3-dimethylbut-1-ene.**

(57) 1,2-Dichloro-3,3-dimethylbut-1-ene is prepared by heating 1,1,2-trichloro-3,3-dimethylbutane in a polar aprotic solvent, preferably in the presence of an alkali or alkaline earth metal halide such as lithium chloride.

EP 0 010 855 A1

ACTORUM AG

COMPLETE DOCUMENT

This invention relates to a process for the manufacture of 1,2-dichloro-3,3-dimethylbut-1-ene.

1,2-Dichloro-3,3-dimethylbut-1-ene has been synthesised from trichloroethylene by reaction with tert-butyl magnesium bromide (J.Normant, Bull. Soc. Chim. France, 1963, Pages 1868-1875), but the latter compound is not readily accessible and is also expensive.

It has now been found that 1,2-dichloro-3,3-dimethylbut-1-ene can be prepared in high yield by selective dehydrochlorination of 1,1,2-trichloro-3,3-dimethylbutane.

According to the present invention there is provided a process for the manufacture of 1,2-dichloro-3,3-dimethylbut-1-ene which comprises heating 1,1,2-trichloro-3,3-dimethylbutane in a polar aprotic solvent.

Examples of polar aprotic solvents which may be used in the process are dimethylacetamide, diethylformamide, hexamethylphosphoramide and especially dimethylformamide.

The rate of reaction may be increased by carrying out the reaction in the presence of an alkali or alkaline earth metal halide, and this is a preferred feature of the invention.

Examples of the alkali and the alkaline earth metal halides which may be used are the iodides, bromides and especially the chlorides of lithium, sodium, potassium and calcium.

1,1,2-Trichloro-3,3-dimethylbutane is readily synthesised from cheap and easily available precursors, namely 1,2-dichloroethylene and tert-butyl chloride, using the method described by L.Schmerling in J.Amer. Chem. Soc., 68, 1655 (1946).

The reaction is carried out at a temperature from 50 to 300°C, preferably 100 to 200°C, under superatmospheric pressure if necessary, and for a reaction time of 0.1 to 100 hrs.

The amount of alkali or alkaline earth metal halide which is used may be from 0.01 to 10.0 mol, preferably 0.10 to 1.0 mol, per mol of 1,1,2-trichloro-3,3-dimethylbutane.

The polar aprotic solvent may be used in an amount from 40 to 5000% by weight based on the weight of 1,1,2-trichloro-2,2-dimethylbutane.

The product may be isolated from the reaction mixture by conventional methods or, for example, by diluting the mixture with an organic solvent which is miscible with the polar aprotic solvent but immiscible with water, washing the diluted mixture with water to remove the polar aprotic solvent and inorganic salts, drying the organic layer, removing the organic solvent by distillation and fractionally distilling the residue. A suitable organic solvent for this method of isolation is methylene chloride.

That 1,2-dichloro-3,3-dimethylbut-1-ene can be prepared by dehydrochlorination of 1,1,2-trichloro-3,3-dimethylbutane is surprising, because it would be expected that the proton most readily lost from the latter compound would be that on the carbon atom bearing two chlorine atoms, and that the product of dehydrochlorination would be 1,1-dichloro-3,3-dimethylbut-1-ene:-

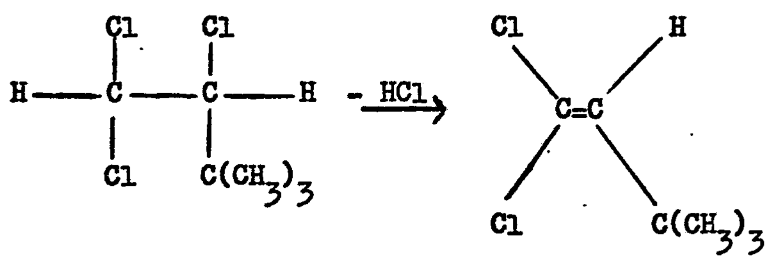


and this does in fact occur exclusively when dehydrochlorination is carried out using potassium hydroxide in diethylene glycol according to the method of Brändstrom in Acta. Chem. Scand., 13, 610 (1959).

The possibility that the process of the invention proceeds by initial formation of the expected 1,1-dichloro-3,3-dimethylbut-1-ene followed by isomerisation thereof to 1,2-dichloro-3,3-dimethylbut-1-ene can be discounted, because heating of the 1,1-dichloro compound in dimethylformamide containing lithium chloride causes no isomerisation to the 1,2-dichloro compound.

Pyrolysis of 1,1,2-trichloro-3,3-dimethylbutane gives a mixture of 1,1- and 1,2-dichloro-3,3-dimethylbut-1-ene which is however virtually impossible to separate.

The method of the present invention gives 1,2-dichloro-3,3-dimethylbut-1-ene exclusively.

1,2-Dichloro-3,3-dimethylbut-1-ene is useful as an intermediate in the synthesis of certain fungicides and plant growth regulators, such as are described in our German Offenlegungsschrift 2737489.

The invention is illustrated but not limited by the following Example:

**Example 1**

1,1,2-Trichloro-3,3-dimethylbutane (106.7 g 0.56 mol) in dried dimethylformamide (185.0 g) containing anhydrous lithium chloride (9.4 g, 0.22 mol) is heated at 160°C for 6 hours. The reaction mixture is dissolved in methylene chloride (100 ml) and the solution is washed with water (4 x 100 ml). The organic layer is dried and fractionally distilled to provide 1,2-dichloro-3,3-dimethylbut-1-ene (78 g, 0.51 mol), yield 90% based on 1,1,2-trichloro-3,3-dimethylbutane. No 1,1-dichloro-3,3-dimethylbut-1-ene is detectable in the product. The following NMR spectrum is obtained: $\tau$ 3.75, singlet, one proton, vinylic; $\tau$ 8.85, singlet, 8.85 protons, aliphatic. This is entirely consistent with the structure.

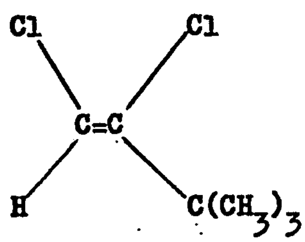

The NMR spectrum of authentic 1,1-dichloro-3,3-dimethylbut-1-ene is similar to the above except that the vinylic proton appears at $\tau$ 4.05.

Example 2

This is a comparative Example illustrating the dehydrochlorination of 1,1,2-trichloro-3,3-dimethylbutane using potassium hydroxide in diethylene glycol.

Potassium hydroxide (0.56 g, $10^{-2}$ mol) in diethylene glycol (3 ml) is warmed to 140°C. The 1,1,2-trichloro-3,3-dimethylbutane (1.9g, $10^{-2}$ mol) is added portionwise during 30 minutes. The heating of the reaction mixture is continued,at 160°C, for 6 hours. The mixture is poured into water and extracted with chloroform.GLC analysis of the extract using a 1 metre Column of 5% E 301 at 160°C showed that the sole dehydrochlorination product is 1,1-dichloro-3,3-dimethylbut-1-ene.

Pyrolysis of 1,1,2-trichloro-3,3-dimethylbutane in the gas phase at 520°C with a residence time of 20 seconds in a flow system gives a mixture of 1,2-dichloro-3,3-dimethylbut-1-ene and 1,1-dichloro-3,3-dimethylbut-1-ene in the ratio 3:1.

Example 3

This is not an example of the present invention but illustrates the attempted isomerisation of 1,1-dichloro-3,3-dimethylbut-1-ene to the 1,2-dichloro compound.

1,1-Dichloro-3,3-dimethylbut-1-ene (8.85 g, 0.056 mol) in dimethylformamide (18.5 g) containing lithium chloride (0.94 g, 0.022 mol) is heated at 160°C for 6 hours. No 1,2-dichloro-3,3-dimethylbut-1-ene could be detected at the end of this period by NMR analysis of the isolated product.

## CLAIMS

1. A process for the manufacture of 1,2-dichloro-3,3-dimethylbut-1-ene characterised in that 1,1,2-trichloro-3,3-dimethylbutane is heated in a polar aprotic solvent.
2. A process as claimed in Claim 1 characterised in that the polar aprotic solvent is dimethylformamide.
3. A process as claimed in Claim 1 or Claim 2 characterised in that the reaction is carried out in the presence of an alkali or alkaline earth metal halide.
4. A process as claimed in Claim 3 characterised in that the alkali or alkaline earth metal halide is the chloride of lithium, sodium, potassium or calcium.
5. A process as claimed in Claim 3 or Claim 4 characterised in that the amount of alkali or alkaline earth metal halide which is used is from 0.01 to 10.0 mols per mol of the 1,1,2-trichloro-3,3-dimethylbutane.
6. A process as claimed in Claim 3 or Claim 4 characterised in that the amount of alkali or alkaline earth metal halide which is used is from 0.1 to 1.0 mol per mol of the 1,1,2-trichloro-3,3-dimethylbutane.
7. A process as claimed in any one of Claims 1 to 6 characterised in that the amount of polar aprotic solvent which is used is from 40 to 5000 per cent by weight based on the weight of 1,1,2-trichloro-3,3-dimethylbutane.
8. A process as claimed in any one of Claims 1 to 7 characterised in that the reaction is carried out at a temperature from 50 to 300°C.
9. A process as claimed in any one of Claims 1 to 7 characterised in that the reaction is carried out at a temperature from 100 to 200°C.

0010855

European Patent Office

# EUROPEAN SEARCH REPORT

Application number
EP 79 30 1986

| Category | Citation of document with indication, where appropriate, of relevant passages | Relevant to claim | CLASSIFICATION OF THE APPLICATION (Int. Cl. 3) |
|---|---|---|---|
| A | JOURNAL OF THE AMERICAN CHEMICAL SOCIETY, vol. 71, June 1949, pages 2015-2019 Easton, U.S.A. L. SCHMERLING et al.: "Peroxide-induced Condensation of Saturated Hydrocarbons with Polychloro-ethylenes" * Page 2017, right-hand column - page 2018, left-hand column * -- | 1 | C 07 C 21/04 17/34 |
| A,D | ACTA CHEMICA SCANDINAVICA, vol. 13, 1959, pages 610-611 Copenhagen, DK. A. BRANDSTROM: "A new Method to prepare Trialkylacetic Acids" * Page 610, right-hand column * ---- | 1 | TECHNICAL FIELDS SEARCHED (Int.Cl. 3) C 07 C 17/34 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

[X] The present search report has been drawn up for all claims

| Place of search | Date of completion of the search | Examiner |
|---|---|---|
| The Hague | 23-01-1980 | VAN GEYT |

EPO Form 1503.1 06.78